# EUROPEAN PATENT APPLICATION

(11) **EP 3 254 717 A1**
(43) Date of publication of application: **13.12.2017**
(21) Application number: 16173802.6
(22) Date of filing: 09.06.2016
(51) Int. Cl.: A61M 5/31, A61M 5/315

(54) **SYRINGE AND METHOD FOR DISPENSING A LIQUID**

(71) Applicant: NOVARTIS AG, 4056 Basel (CH)
(72) Inventor: LATHAM, David, 4056 Basel (CH); BRYANT, Andrew, 4056 Basel (CH); MARSHALL, Steve, Mansfield, Nottinghamshire NG19 8RL (GB); WESSELTOFT MOGENSEN, Lasse, St. Ives, Cambridgeshire PE27 6SB (GB); STEIN, Andrew John, Jamaica Plain, MA 02130 (US)
(74) Representative: Viering, Jentschura & Partner mbB Patent- und Rechtsanwälte

(57) **Abstract**

The present invention relates to a syringe for dispensing a liquid, the syringe (1) comprising: a barrel (10) including a first end (12) and a second end (14) opposite the first end (12), wherein said barrel (10) is provided with a discharge opening (18) at or near said second end (14) and wherein at least one first chamber (26) is defined inside said barrel (10), with said first chamber (26) for receiving said liquid to be dispensed through said discharge opening (18), wherein a plunger (28) is received within said barrel (10), wherein said plunger (28) supports an element (30) that is both translationally movable with regard to the barrel (10) and translationally movable with regard to the plunger (28), wherein the movement of aid element (30) is controllable independently from the movement of said plunger (28), and to a method.

## Description

The present invention refers to a syringe and a method for dispensing a liquid out of the primed syringe, particularly for priming a syringe and subsequent dosing of a predetermined dose of said liquid.

Some conventional syringes may be pre-filled with liquid.

However, conventional syringes are, nevertheless, still widespread. Conventional syringes need to be filled by sucking a liquid drug into said syringe, prior to dispensing the liquid fluid and prior to injecting said liquid fluid into the skin of a patient. A standard type of a conventional syringe comprises one plunger including one piston head and one piston rod attached to said piston head, wherein said piston head is inserted into the inner space of a barrel. The piston rod extends from an outside of said barrel to said piston head, and thus enables the user to operate a displacement of the piston head within the barrel from outside the barrel. The barrel has a first end and a second end opposite the first end. While the piston rod extends out of the barrel at the first end, the second end of the barrel is provided with a discharge opening open to a first chamber of the syringe, which chamber is limited by the piston head and provided for receiving the liquid drug.

Prior to dispensing a liquid fluid contained in a conventional syringe and prior to injecting a liquid fluid contained in the conventional syringe, respectively, such kind of syringes must be primed. Priming may involve sucking of a liquid drug into said syringe (see above). In addition, priming may involve removing air bubbles and/or liquid from the first chamber, respectively. If such air bubbles are still present in the first chamber upon start of injection, there is the danger that air is injected to the patient which may be dangerous and painful for the patient, and may reduce the volume of liquid dispensed which may reduce the intended benefit of the injection.

For the purpose of priming, the user may pull the piston rod of a standard type of a conventional syringe in order to suck a liquid drug through the discharge opening.

The user may then rotate the syringe such that the second end is directed upwards. The difference in the density between the liquid and the air will then mean that the gravitational forces will pull the liquid to the first end of the syringe, thus displacing the air to the second end. However, this process may be impeded by resistive forces which may be influenced by the diameter of the syringe and/or by the surface tension of the liquid. To overcome these resistive forces the user may apply additional acceleration/vibration to the syringe. Common methods of generating such syringe acceleration/vibration while maintaining the correct orientation may be for the user to use their finger to flick the side of the syringe barrel. Once the liquid and the air have been correctly separated into the desired ends of the syringe, the user may slightly push the piston rod towards the second end to move the air and any excess liquid out of the of the syringe. After that, injection may start.

Such kind of syringes are available on the marked in various sizes, i.e. differing by the maximum volume of their first chambers and/or differing by the diameter of their piston heads. Accordingly, the smaller the dose to be injected into a patient, the smaller the size of the syringe and diameter of the piston head (and/or barrel), respectively, should or can be selected to reduce the variation of the dispensed volume due to dose mark alignment variation.

It is often desirable to deliver a relatively small volume of fluid from a syringe in a method that is both accurate and reproducible. However, it is difficult to deliver accurately a relatively small volume of fluid during the delivery step using a conventional syringe, wherein it is ensured that the respective fluid is free of air bubbles.

Particularly, the above-mentioned methods of "rotating the syringe" and/or using a "syringe acceleration/vibration generated by a commonly used method" and/or "slightly pushing the piston rod thereafter" may not work, if the diameter of the barrel of a standard conventional syringe becomes too small. However, using larger diameters of the barrel of a standard conventional syringe implies larger piston head diameters, which makes it more difficult to exactly dose the fluid drug to be dispensed as the influence of syringe diameter and associated resistive forces.

Some further developed syringes are disclosed in each of WO 01/62319, US 3,934,586 and WO 03/004080, each of which describes dual stage syringes, but none of them addresses the issue of delivering accurately and reproducibly a relatively small volume of a fluid.

According to a first aspect of the present invention, a syringe for dispensing a liquid as defined in any of claims 1 or 11 is provided. A method according to the present invention is defined in claim 14. The dependent claims show some examples of such a syringe and method, respectively. The invention particularly provides a syringe for dispensing a liquid, with the syringe comprising a barrel, a plunger, and an element that is both translationally movable with regard to the barrel and translationally movable with regard to the plunger, particularly translationally movable with regard to the plunger in the longitudinal direction of the barrel.

Preferably, the liquid is a liquid drug.

Said element supported by said plunger may be a rod.

It must be noted, however, that said above-mentioned movability of said element does not exclude that said element may be blocked, particularly releasably blocked, and/or stopped with regard to the barrel and/or plunger. To the contrary, in some preferred embodiments of the present invention, there is provided a stop and/or locking device for the element, like a rod, in at least one, especially in two, distanced axial positions of the rod, wherein the distance of these axial positions is such that moving the rod from a first of these two positions to the second of these two positions corresponds to a predetermined dose to be dispensed by the syringe. In other words, the syringe is provided with a first chamber provided for receiving a liquid (see below), wherein the volume of said first chamber, which volume is available for receiving said liquid, is smaller in the second position than in the first position of the rod, and this difference in volume is caused be the difference in positions of the rod (i.e. all other parameters, like position of the plunger may be identical). Said difference in volume may correspond to the dose to be dispensed.

According to the present invention, the barrel includes a first end and a second end opposite the first end. Said barrel is provided with an opening and/or discharge opening and/or a passage and/or discharge passage (in short: discharge opening) at or near said second end, and at least one first chamber (already mentioned above) is defined inside said barrel. Said first chamber is configured to be filled with said liquid to be dispensed through said discharge opening at a later stage, i.e. after it has been filled into said first chamber.

A plunger is receivable and received within said barrel, respectively. In particular, the plunger may be slidably received within said barrel.

The plunger may be provided to allow priming of said syringe in a simple manner, even if the dose to be dispensed is very small.

Priming may include and/or consist of filling the first chamber with a liquid through said discharge opening or discharge passage into said first chamber and/or removing gas or air bubbles contained in said first chamber out of the first chamber and liquid, respectively, and/or moving to a starting positon for starting dispensing, like to a (first) stop position (see below). Priming may be done by displacing said plunger.

Said plunger supports an element, like rod, that is both translationally movable with regard to the barrel and translationally movable with regard to the plunger, wherein movement said element (for example rod) is controllable from a position outside of the barrel and/or is controllable independent from said plunger and independently from movement of said plunger, respectively. Said element (for example rod) supported by said plunger may be a rod. For the ease of reference to this member, said element (for example rod) supported by said plunger will be named 'rod' in the following, wherein it must be noted that a rod is only one example for such an element, so that other kinds of such elements supported by said plunger may be applied instead of said exemplary rod.

Particularly, the volume presently available in the first chamber for receiving said liquid may be changeable by means of said plunger displacement. Particularly, the volume presently available in the first chamber for receiving said liquid is changeable by means of said rod in order to allow dosing and/or displacement of liquid out of said syringe.

In some embodiments, the movement of said rod may be mechanically controllable. In addition to that or as an alternative, movement of said rod may be electronically and/or electro-magnetically controllable.

The length of said rod may be larger than the length of said plunger.

Said plunger may be provided with a through hole, wherein said rod may slidably pass through said through hole into said first chamber.

The rod may close, for example sealingly close, said through hole provided in the plunger and/or provided in both a piston head of said plunger and a piston rod of said plunger, particularly in any position of the plunger, upon priming of said syringe and/or upon dispensing liquid through said discharge opening.

Said plunger may be provided with both a piston head and piston rod. Both the piston head and piston rod may be provided with a through hole extending in longitudinal direction thereof. Said through hole of said piston head and said through hole of said piston rod may be connected to each other. Said through hole of said piston head and said through hole of said piston rod may have circular cross sections, wherein the cross section may be of constant size. In addition the through hole of said piston head and said through hole of said piston rod may form a single through hole of the plunger. The central axis of said through holes may in parallel to and/or coincide with the central longitudinal axis of the barrel.

The barrel may have a cylindrical shape.

Said piston rod may be hollow and the rod may extend through said piston rod.

The piston head may be of any sealing material, for example. The rod may extend through the piston head in a sealing manner.

Said plunger and said rod may be configured such that longitudinal displacement of the plunger by a predetermined length towards the first end of the barrel (i.e., in the first direction) enlarges the volume of the first chamber to a greater extent than longitudinal displacement of said rod by the same predetermined length towards said second end of the barrel (i.e., in the second direction) reduces the volume of said first chamber.

The first chamber may be delimited by the piston head of the plunger, on the one hand, and by an end wall of the barrel at the second end of the barrel, on the other hand. In radially outer direction, the first chamber may be delimited by the barrel.

When seen in radial direction of said barrel and rod, respectively, the piston head may extend from said barrel to said rod. Alternatively, the piston head may be interrupted by said piston rod such that a first member of said piston head extends from said barrel to the radially outer surface of said piston rod, and a second member of said piston head extends from the radially inner surface of said piston rod to said rod, seen in radial direction of said barrel and rod, respectively. Both said first member of said piston head and said second member of said piston head may be made from a sealing material. Both said first member of said piston head and said second member of said piston head may be designed ring-shaped.

The piston head and the first and second members of the piston head, respectively, may be made of another material than the piston rod, but be connected to the piston rod. As mention above, a sealing material is preferred for the piston head and the first and second members of the piston head, respectively.

The above movability of the rod and/or the plunger particularly refers to the movability along the longitudinal axis of the barrel.

The material of the piston head may be different from the material of the piston rod. The material of the piston head may provide sealing properties in order to seal the plunger against the rod and/or the barrel.

Both the rod and the plunger may be inserted from the first end into the barrel. Both the rod and the plunger may protrude out of the first end of the barrel.

According to a second aspect of the present invention, a syringe for dispensing a liquid is provided, wherein said syringe comprises a barrel, a piston, and a dosing device. Said barrel includes a first end and a second end opposite the first end, and said barrel is provided with a discharge opening at or near said second end. Said piston is provided in said barrel. For example, said piston may be movably arranged within said barrel. In a preferred embodiment, said piston may be lockable with regard to said barrel. At least one first chamber is defined inside said barrel by means of said piston. Said first chamber can be filled with said liquid to be dispensed through said discharge opening. Said dosing device is configured to dose a predetermined volume of liquid contained in said first chamber and said dosing device is configured to dispense said dose through said discharge opening. It must be noted that said dosing device is different from said piston. Said dosing device has at least one state in which it is movable with respect to both said barrel and said piston, i.e. even if the dosing device or a member thereof may be lockable in preferred embodiments with regard to the barrel and/or the piston, there is at least one stage which it is an unlocked stage so that the dosing device or a member thereof may move relative to the barrel and/or relative to the piston.

It must be noted that the first aspect of the present invention may also be combined with the second aspect of the present invention. Besides, preferred embodiments of the first aspect of the present invention may also represent preferred embodiments of the second aspect of the present invention, and preferred embodiments of the second aspect of the present invention may also represent preferred embodiments of the first aspect of the present invention.

In this respect, the piston of the second aspect of the present invention may correspond to the plunger of the first aspect of the present invention. The piston may be a floating piston. In another preferred embodiment of the present invention, the piston may have a piston head and a piston rod.

A first position of said element supported by said plunger and/or of said rod and/or of said dosing device may be defined by means of a first stop and/or by means of a first locking device for said element and/or for said rod and/or for said dosing device, and a second position of said element supported by said plunger and/or of said rod and/or of said dosing device may be defined by means of a second stop and/or by means of a second locking device for said element supported by said plunger and/or for said rod and/or for said dosing device, wherein the distance between said second position and said first position, in combination with the cross sectional area of said element (rod) defines said dose, i.e. predetermined volume of the liquid to be dispensed, considering the shape and/or dimensions of said element supported by said plunger and/or rod and/or said dosing device.

The respective first and second positions may be positions of the rod relative to said plunger and/or relative to said barrel.

A third stop and/or a third locking device may be provided for blocking or for locking the rod to the plunger. The respective blocked or locked position is preferably selected, respectively, when the dose to be dispensed is started to be sucked into the first chamber.

A fourth stop and/or a fourth locking device may be provided for locking the plunger and piston, respective, to barrel. The respective locked position is preferably selected, respectively, when the dose to be dispensed is dispensed.

A first locking device may be releasable. A second locking device may be releasable. A third locking device may be releasable. A fourth locking device may be releasable.

According to a preferred embodiment of the present invention, there is provided a (first) locking device for locking the rod to the plunger. Said (first) locking device may be releasable and may be arranged such that after release of (said first) locking device the rod may move relative to the plunger in the direction of the second end of the barrel. Preferably, the barrel, for example the rearward end (= first end) of the barrel (i.e. the first end of the barrel), provides a (third) stop for the plunger. For example the plunger may have a flange at its rearward end, which may come in contact with the rearward end of the barrel, thus blocking further motion of the plunger relative to the barrel in the direction of the second end of the barrel. Having in mind that the rod may be held on the plunger by means of the (first) locking device, said stop position may define as a starting position for sucking liquid into the syringe. Preferably, the plunger, for example the rearward end of the plunger, provides a (second) stop for the rod. For example, the rod may have a flange at its rearward end, which may come in contact with the rearward end of the plunger, thus blocking further motion of the rod relative to the plunger in the direction of the second end (forward end) of the barrel. The latter stop positon may define the second position of the rod. The distance between said first and second positions of the rod (in combination with the cross sectional area of said rod) may define a dose to be dispensed by moving the rod, with the plunger being held in position with respect to the barrel.

The syringe and discharge opening and discharge passage, respectively, may be provided with a needle.

According to a third aspect of the present invention, a syringe for dispensing a fluid is provided, the syringe comprising a barrel including a discharge end defining a discharge passage, and a plunger disposed within the barrel, the plunger being adapted to move within the barrel such that the plunger and discharge end define a variable volume chamber within the barrel and the plunger is capable of displacing fluid from the chamber through the discharge passage, wherein a translational element provided, which translational element is adapted to move about a longitudinal axis of the plunger and which has a piston head in an orientation relative to the discharge end of the barrel and wherein the translational element can be moved separately from and independently of the movement of the plunger, respectively. The translational element may correspond to said element supported by said plunger and be identical to said element, respectively.

It must be noted that the first aspect of the present invention and/or the second aspect of the present invention may also be combined with the third aspect of the present invention.

Besides, preferred embodiments of the first aspect of the present invention and/or preferred embodiments of the second aspect of the present invention may also represent preferred embodiments of the third aspect of the present invention, and preferred embodiments of the third aspect of the present invention may also represent preferred embodiments first and/or second aspects of the present invention.

The translational element may be adapted to be moved between a first and second position by a driving mechanism.

The plunger may include a piston located within the barrel, an elongate shaft and piston rod, respectively, extending from the piston and a push button at the end of the shaft opposite to the piston and piston head, respectively.

The plunger shaft (piston rod) may have a cruciform cross sectional configuration and each arm of the cruciform shaft may define a notch such that all of the notches are equally spaced from the push button.

Movement of the translational element may be limited by the interaction of the piston head at a surface of the discharge end.

The push button at the end of the shaft opposite to the piston may carry the driving mechanism for the translational element.

The translational element may have a piston rod and the piston head.

In accordance with an embodiment of the invention, the plunger can be displaced towards the discharge end (second end) in the second axial orientation until the first stop is engaged. This may constitute the constitution of the priming step. The translational element may then be moved from a first position to a second position and the spacing between the first and second stops may define the volume dispensed from the syringe during the delivery step for a specific barrel internal diameter. For relatively small volumes of fluid (liquid or liquid drug, for example), the displacement from the first position to the second position is relatively short, but accurately controlled by the syringe according to the invention.

The translational element may be adapted to be moved between the first and second positions by a driving mechanism.

A common arrangement for plungers may include a cruciform-shaped shaft (piston rod). Thus, in an embodiment of the invention as defined anywhere herein, the plunger may include a piston located within the barrel, an elongate shaft extending from the piston, and a push button at the end of the shaft opposite to the piston, wherein the plunger shaft may have a cruciform cross sectional configuration and each arm of the cruciform shaft may define a notch such that all of the notches are equally spaced from the push button.

In a further embodiment of the invention, the barrel may include a collar at the opposite end to the discharge end, and one of the collar and the plunger may include an axial projection and the other of the collar and the plunger may include a first stop surface and a recess sized and configured to receive therein the projection, wherein the projection is adapted to align axially with the first stop surface in the second orientation of the plunger and the projection is adapted to align axially with the recess in the first axial orientation, whereby the plunger movement is limited in the second orientation by the interaction of the projection and the first stop surface and further movement of the plunger is permitted in the first orientation by the projection being received into the recess.

In this embodiment, the maximum displacement of the translational element into the recess may be defined by the distance between the end position of the piston of the plunger and the discharge end of the barrel.

A method according to the present invention provides a method for dispensing a liquid out of said syringe, wherein said syringe comprises a barrel provided with a discharge opening and a piston and/or plunger received within said barrel, wherein the method comprises the step of dosing and dispensing a predetermined volume of a liquid of liquid contained in the first chamber, wherein said piston and/or plunger is hold in position with respect to said barrel upon dosing and/or dispensing.

According to a preferred embodiment of said method, said predetermined volume is predetermined by means of a first stop and/or by means of a first locking device, on the one hand, and by means of a second stop and/or by means of a second locking device, on the other hand.

A syringe according to the present invention may be used for performing the method according to the present invention.

An exemplary embodiment of the invention will now be described with reference to the accompanying drawings in which:
- Fig. 1: shows a view of an exemplary syringe according to the invention.
- Fig. 2: shows the embodiment of Fig. 1, with both the plunger and the both the plunger and the element (rod) supported by the plunger further moved towards the second end of the barrel, when compared to the position of Fig. 1,
- Fig. 3: shows the embodiment of Figures 1 and 2, with both the plunger and the element (rod) supported by the plunger further moved towards the second end the barrel, when compared to the positions of Figures 1 and 2, wherein the rod is in a position on a fourth stop (not shown, but shown in figure 5);
- Fig. 4: shows the embodiment of Figures 1 through 3, with both the plunger in the same position as shown in Fig. 3, and with the element (rod) supported by the plunger further moved towards the second end the barrel and being in a second stop position.

For the avoidance of doubt, the skilled person will appreciate that in this specification, the terms "up", "down", "front", "rear", "upper", "lower", "width", etc. refer to the orientation of the components as found in the syringe when installed for normal use as shown in the Figures.

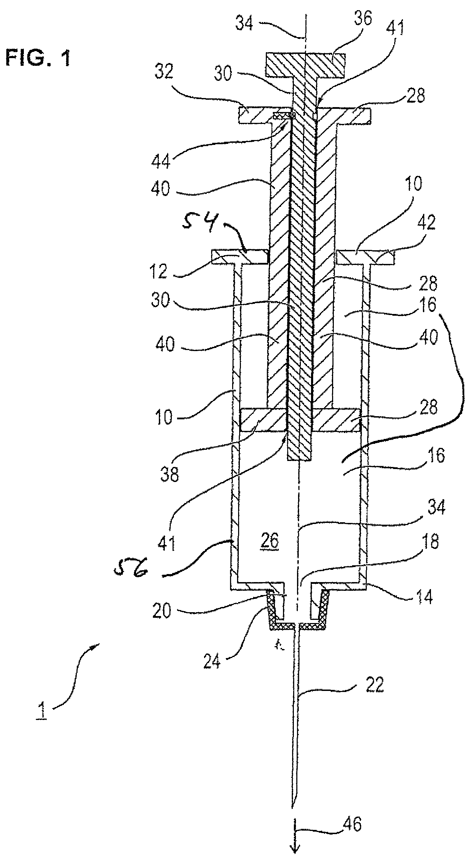

Fig. 1 shows a syringe 1 for dispensing a liquid.

The syringe 1 comprises a barrel 10 including a first end (rearward end) 12 and a second end (forward end) 14 opposite the first end 12. The barrel 10 is of a cylindrical shape defining an inner space 16.

The barrel 10 is further provided with a discharge opening 18 and discharge passage 20, respectively, to which a needle 22 can be connected.

Secured to the discharge end (second end) 14 of the barrel 10 is a connecting collar 24 of a hypodermic needle 22.

The skilled person will appreciate that the hypodermic needle connecting collar 24 may be a simple friction fit with the discharge end 14 of the barrel 10, or it may be adhered to the discharge end 14 of the barrel 10, or it may be a locking collar, such as a Luer-Lock collar. The connection of hypodermic needles 22 to syringe barrels 10 is well known in the art and will not be described in detail herein. The invention can, however, also used without a needle or with another type of needle or fixing device for a needle.

A first chamber 26 is defined inside said barrel 10 and forms a part of the inner space 16, respectively. Said first chamber 26 can be filled with a liquid to be dispensed through said discharge opening 18 at a later stage.

A plunger 28 is received within said barrel 10, wherein said plunger 28 supports an element 30 that is both translationally movable with regard to the barrel 10 and translationally movable with regard to the plunger 28, wherein movement said element 30 is controllable from outside of the barrel 10 and/or is controllable independent from said plunger 28 and independently from movement of said plunger 28, respectively. For the purpose of realizing the latter, the element 30 extends to the outside of the barrel 10 in this example, wherein element 30 is a rod 30 in this example.

Plunger 28 comprises a flange 32 on its rearward end and at the rearward end to the piston rod 40, respectively, which extends perpendicular to the longitudinal axis 34 of the plunger 28 and rod 30 and barrel 10, respectively. Flange 32 may be ring-shaped. Piston rod 40 may have a cruciform cross section which terminates in said flange 32 at rearward end of the piston rod, such that the rearward facing surface of the flange 32 forms a pull and push surfaces for the plunger 28.

In the direction of the longitudinal axis 34, the rod 30 is longer than the plunger 10.

Rod 30 comprises a flange 36 on its rearwards end, which extends perpendicular to the longitudinal axis 34 of the plunger 28 and the rod 30 and the barrel 10, respectively. Flange 36 may have a circular shape.

Plunger 28 comprises a piston head 38 and a piston rod 40, with the diameter of the piston head 38 being larger that the diameter of the piston rod 40. In an alternative embodiment the diameter of the plunger 28 may be constant, apart from the flange 36.

Plunger 30 is provided with a through hole 41 that extends through the piston rod 40 and through the piston head 38 (and through the flange 32, if present) in the direction of the longitudinal axis 34. Rod 30 extends through said through hole 41.

Barrel 10 comprises a flange 42 on its rearwards end (first end) 12 which extends perpendicular to the longitudinal axis 34. Flange 42 may be ring-shaped.

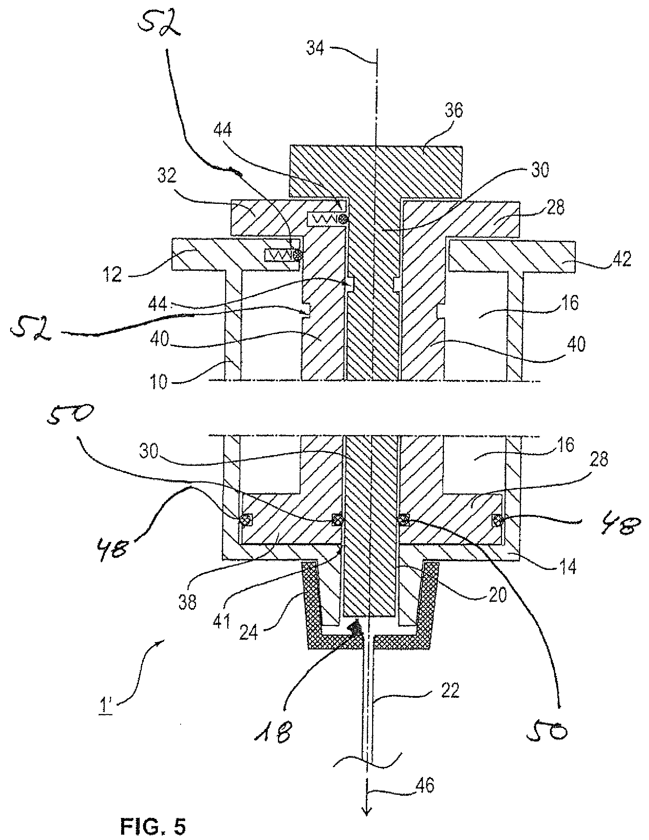

Syringe 1 comprises a (first) locking device 44 for locking the rod 30 to the plunger 28. Said (first) locking device 44 may is releasable and is arranged such that after release of (said first) locking device 44, the rod 30 may move relative to the plunger 28 in the direction of the second end 14 of the barrel 10. Locking device 44 may prevent movement of the translational element (rod) 30 relative to the plunger 28 before the fourth stop (not shown, but shown in Fig. 5) is reached. This ensures that the translational element remains in the position relative to the plunger 28 until the priming step is completed and the plunger has engaged a fourth stop.

The rearward end (= first end) 12 of the barrel 10 provides a (third) stop, which is a stop for the plunger 28 and via the plunger 28 and the locking device 44 is a releasable stop for the rod 30, respectively. In this respect, the flange 32 may come into contact with the flange 42, thereby blocking further motion of the plunger 28 in the direction towards the second end 14 relatively to the barrel 10.

The rearward end of the plunger 28 and the flange 32, respectively, provide a (second) stop, which is a (second) stop for the rod 30. In this respect, the flange 36 may come into contact with the flange 32, thereby blocking further motion of the rod 30 in the direction towards the second end 14 relatively to the plunger 28.

When the rod 30 is held in position at or on the plunger 28 by means of the (first) locking device 44, said stop position at the first stop may define a first position of the rod 30, and said stop position at the second stop may define a second position of the rod 30.

By means of the various positions and states of the syringe 1 shown in figures 1 to 4, respectively, an exemplary operation of an exemplary embodiment of the syringe according to the present invention, and an exemplary embodiment of the method according to the present invention will be described:

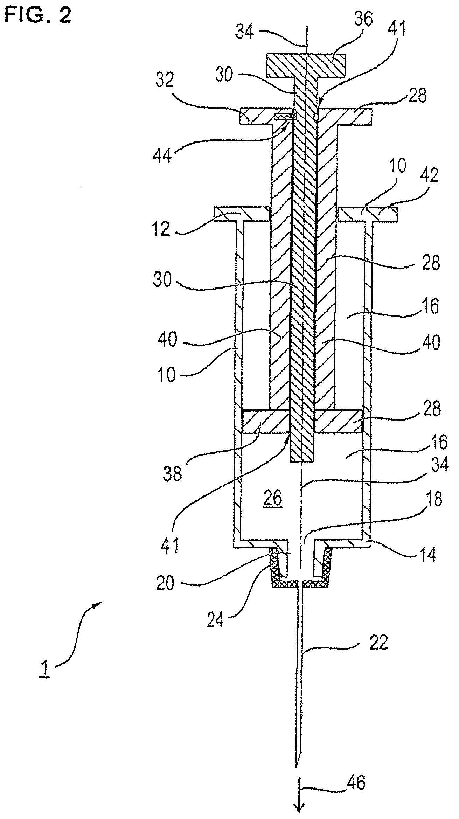

When compared to Figures 2 to 4, Fig. 1 show both plunger 28 and rod 30 in a maximum retracted position, where the locking device 44 is in engagement so that the movement of the plunger 28 causes respective movement of the rod 30. The plunger 28 and the rod 30, connected by the locking device 44 to each other, may have been moved to said position from a fully inserted position (i.e. combined unit of plunger 28 and rod 30 (for example maximum) inserted into barrel 10) in order to suck a liquid through the discharge opening 18 into the first chamber 26.

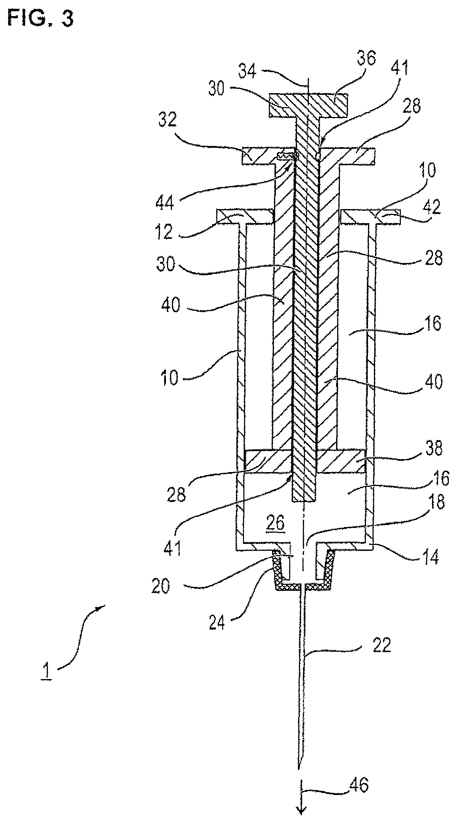

Figure 2 shows a position and state of the syringe 1, respectively, that may be passed upon sucking a liquid through the discharge opening 18 into the first chamber 26, as well as upon moving in opposite direction towards the positon and state of the syringe 1, respectively, which is shown in Fig. 3. In Fig. 2 the locking device 44 is in engagement.

Figure 3 shows a position and state of the syringe 1, respectively, in which the first stop 44 is reached. Particularly, the flange 32 of the plunger 28 is in contact with the flange 36 of th ebarrel 10, and the rod 30 is thus also in a stop position, since the locking device 44 holds the rod 30 in position with regard to the plunger 28.

Upon movement from the position shown in Fig. 1 to the position shown in Fig. 3, superfluous liquid as well as air can be removed from the first chamber thanks to the design not requiring very small diameters of the barrel 10, but allowing dispensing very small volumes thanks to the rod 30 in this exemplary embodiment.

By further pushing the rod 30 in the second direction (indicated by arrow 46) and applying a respective force sufficient to overcome the holding force of the locking device 44 (for example, locking device may have a recess in combination with spring biased ball engaging said recess), the rod 30 continues movement in the second direction. It must be noted that other locking devices 44 may be used in the present invention, which might, for example, require actuating a release member instead of applying a force higher that a holding force.

Upon this continued movement of the rod in the second direction, the intended dose of liquid fluid is dispensed.

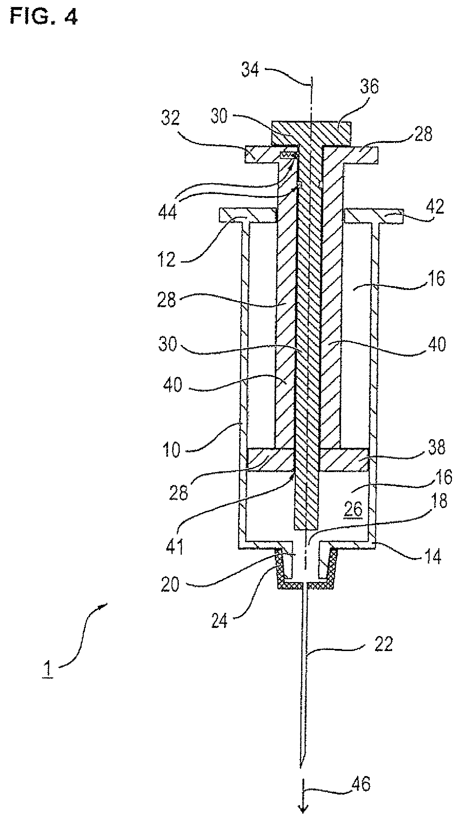

In order to ensure exactly the predetermined dose to be dispensed, a second (stop position) of the rod 30 is provided, as shown in Fig. 4.

This is exemplary realized in this embodiment by providing a stop 32, 42 for the rod 30 and the flange 36 of the rod 36, respectively, for example by means of the flange 32 being in contact with a blocked by flange 42 with regard to movements in the second direction.

Figure 5 shows some further exemplary features (particularly first, second, third, and fourth further features) that may be singly or in any combination used in the embodiment as shown in figures 1 to 4, and in a respective method, respectively.

As a first further feature, Fig. 5 shows a first seal 48 sealing the piston head 38 against the inner circumferential wall of the barrel 10. Said first seal 48 may be received in a first groove provided in the outer circumference of said piston head 38.

As a second further feature, Fig. 5 shows a second seal 50 sealing the piston head 38 and/or plunger 28, respectively, against the outer circumferential wall of the rod 30. Said second seal 50 may be received in a second groove provided in the inner circumference of said piston head 38 and plunger 28, respectively.

As a third further feature, Fig. 5 shows a fourth stop and locking device 52, respectively, for stopping and/or releasably locking plunger 28 and piston, respectively, to the barrel 10.

As a fourth further feature, Fig. 5 shows that the discharge opening 18 defines a second chamber. The outer dimensions of the second chamber may substantially correspond to the outer dimensions of the facing end section of the rod 30 so that the rod 30 can enter the second chamber.

For example, the invention may be used for delivering of an ophthalmic solution (liquid).

Moreover, the syringe may be provided with dose marks.

It must be noted that the end plate 54 of barrel 10 may be attached to the outer shell 56 of the barrel 56 after the plunger 28 has been inserted into the outer shell of the barrel 10, in order to allow mounting the syringe. However, there are alternative options, like diving the outer shell 56 into two separate parts to be attached to each other after the piston rod 40 has passed the through hole 41 and, thereafter, the piston rod has been attached to the piston head 38, or the like. In any case, preferably, the barrel is made to be one piece after the plunger 28 has passed the hole 41.

### Reference numerals

- 1: syringe
- 10: barrel
- 12: first end (rearward end) of 10
- 14: second end (forward end) of 10
- 16: inner space of 10
- 18: discharge opening in 10
- 20: discharge passage
- 22: needle
- 24: connecting collar of 22
- 26: first chamber
- 28: plunger
- 30: element supported by 28, rod
- 32: flange of 28
- 34: longitudinal axis of 10, 28, 30
- 36: flange of 30
- 38: piston head
- 40: piston rod
- 41: through hole in plunger
- 42: flange of 10
- 44: first locking device
- 46: second direction
- 48: first seal
- 50: second seal
- 52: fourth locking device
- 54: end plate of 10
- 56: outer shell of 10

## Claims

1. A syringe for dispensing a liquid, the syringe (1) comprising: a barrel (10) including a first end (12) and a second end (14) opposite the first end (12),
wherein said barrel (10) is provided with a discharge opening (18) at or near said second end (14) and wherein at least one first chamber (26) is defined inside said barrel (10), with said first chamber (26) for receiving said liquid to be dispensed through said discharge opening (18), wherein a plunger (28) is received within said barrel (10), wherein said plunger (28) supports an element (30) that is both translationally movable with regard to the barrel (10) and translationally movable with regard to the plunger (28), wherein the movement of said element (30) is controllable independently from the movement of said plunger.

2. The syringe according to claim 1, wherein said element (30) supported by said plunger (28) is a rod (30).

3. The syringe according to any of the preceding claims, wherein the length of said rod (30) is larger than the length of said plunger (28).

4. The syringe according to any of the preceding claims, wherein said plunger (28) is provided with a through hole (41), wherein said element (30) supported by said plunger (28) extends through said through hole (41) into said first chamber (26).

5. The syringe according to any of the preceding claims, wherein said plunger (28) is provided with both a piston head (38) and piston rod (40).

6. The syringe according to claim 5, wherein the piston rod (40) is hollow and
wherein said rod (30) extends through said piston rod (38).

7. The syringe according to any of the preceding claims, wherein said plunger (28) and/or said piston head (38) and/or said piston rod (40) is in sealing engagement with said element (30) and/or wherein the piston head (38) or piston rod (40) is in sealing engagement with said barrel (10).

8. The syringe according to any of the preceding claims, wherein the plunger (28) and the element (30) supported by said plunger (28) are configured such that longitudinal displacement of the plunger (28) by a predetermined length towards the first end (12) enlarges the volume of the first chamber (26) to a greater extent than subsequent longitudinal displacement of said element (30) supported by said plunger (28) by the same predetermined length towards said the second end (14) reduces the volume of said first chamber (26).

9. The syringe according to any of claims 5 to 8, wherein said piston head (38) extends from said barrel (10) to said rod (30), seen in radial direction of said barrel (10) and rod (30), respectively.

10. The syringe according to any of claims 5 to 9, wherein said piston head (38) is interrupted by said piston rod (40) such that a first member of said piston head (38) extends from said barrel (10) to the radially outer surface of said piston rod (40), and a second member of said piston head (38) extends from the radially inner surface of said piston rod (40) to said rod (30), seen in radial direction of said barrel (10) and rod (30), respectively.

11. A syringe for dispensing a liquid, the syringe (1) comprising:
- a barrel (10) including a first end (12) and a second end (14) opposite the first end (12), wherein said barrel (10) is provided with a discharge opening (18) at or near said second end (14);
- a piston (28, 38, 40) provided in said barrel (10);
wherein at least one first chamber (26) is defined inside said barrel (10) by means of said piston (28, 38, 40), with said first chamber (26) for receiving said liquid to be dispensed through said discharge opening (18), particularly a syringe (1) according to any of the preceding claims, **characterized by** a dosing device (30, 44) configured to dose a predetermined volume of said liquid contained in said first chamber (26) and configured to dispense said dose through said discharge opening (18), wherein said dosing device is different from said piston, and wherein said dosing device has at least one state in which it is movable with respect to both said barrel (10) and said piston (28, 38, 40).

12. The syringe according claim 11 and according to any of claims 1 to 10,
wherein the plunger (28) is the piston, or vice versa, and wherein said element (30) supported by said plunger (28) is a member of said dosing device (30, 44) or is said dosing device (30, 44).

13. The syringe according to any the preceding claims, wherein a first position of said rod (30) and/or a first position of said element (30) supported by said plunger (28) and/or a first position of said dosing device (30, 44) is defined by means of a first stop (44) and/or by means of a first locking device (44) for said rod (30) and/or for said element (30) supported by said plunger (28) and/or for said dosing device (30, 44), and wherein a second position of said rod (30) and/or a second position of said element (30) supported by said plunger (28) and/or a second position of said dosing device (28) is defined by means of a second stop (36, 32) and/or by means of a second locking device for said rod (30) and/or for said element (30) supported by said plunger (28) and/or for said dosing device (30 ,44), and wherein the distance between the second position and the first position defines said dose in connection with the shape and/or dimensions of said rod (3) and/or element (30) supported by said plunger (28) and/or said dosing device and/or rod (30).

14. A method for dispensing a liquid out of said syringe, wherein said syringe (1) comprises a barrel (10) provided with a discharge opening (18) and a piston and/or plunger (28) received within said barrel (10), **characterized by** dosing and dispensing a predetermined volume of a liquid of liquid contained in the first chamber (26), wherein said piston and/or plunger (28) is held in position with respect to said barrel (10) upon dosing and/or dosing and/or dispensing.

15. The method of claim 14, wherein said predetermined volume is predetermined by means of a first stop (44) and/or by means of a first locking device (44), on the one hand, and by means of a second stop (36, 32) and/or by means of a second locking device, on the other hand,
